# EUROPEAN PATENT SPECIFICATION

(11) **EP 0 434 410 B1**
(45) Date of publication and mention of the grant of the patent: **02.11.1994**
(21) Application number: 90313952.5
(22) Date of filing: 19.12.1990
(51) Int. Cl.: C07D 311/66, B01J 23/44

(54) **Catalytic production of coumarin compounds**
Katalytische Herstellung von Cumarin-Verbindungen
Production catalytique de composés de coumarin

(30) Priority: 21.12.1989 JP 333153/89; 24.07.1990 JP 197098/90
(43) Date of publication of application: 26.06.1991
(73) Proprietor: SUMITOMO CHEMICAL COMPANY LIMITED, Osaka-shi, Osaka 541 (JP)
(72) Inventor: Shirafuji, Tamio, Niihama-shi, Ehime (JP); Sakai, Kiyomi, Niihama-shi, Ehime (JP); Okusako, Kensen, Niihama-shi, Ehime (JP)
(74) Representative: Diamond, Bryan Clive

(56) References cited:
- US-A- 3 936 473
- CHEMICAL ABSTRACTS, vol. 95, no. 5, August 3, 1981, Columbus, Ohio, USA MILLS,F.D. "Preparation and catalytic hydrogenation of 4,6-dimethyl- and 4,7-dimethyl-8-methoxycoumarins. A 4-methyldihydrocoumarin synthesis" page 733,column 1, abstract -No. 42 822 m
- CHEMICAL ABSTRACTS, vol. 70, no. 5, February 3, 1969, Columbus, Ohio,USAKOZLOV, N.S. et al. "Catalytic synthesis of coumarin derivatives of beta-
- arylamino ketones" page 1974, column 2, abstract -No. 19 866d
- CHEMICAL ABSTRACTS, vol. 108, no. 17, April 25, 1988, Columbus, Ohio,USAMEDZHIDOV, A.A. et al. "Study of the heterogeneous catalytic acylationofphenols in the manufacture of coumarin and chromone" page 723, column 1,abstract- No. 150 239x

## Description

The present invention relates to a process for producing coumarin or a coumarin derivative from a 3-(2-cyclohexanoyl)propionic acid ester derivative.

Coumarin derivatives, along with dihydrocoumarin derivatives formed as by-products, are important compounds particularly in the perfume industry and also as intermediates for agricultural chemicals, medicines and dyes.

Conventionally known methods for producing coumarin derivatives include the process of US-A-3442910 in which a hydrogenation-dehydrogenation catalyst, such as palladium, and a 3-(2-cyclohexanoyl)propionic acid ester derivative as a starting material are first introduced into a reactor, and ring formation and dehydrogenation reaction is then conducted with heating, and the process of JP-A-60/181082 in which the ring formation and dehydrogenation reaction for producing a coumarin derivative is performed in the co-presence of a noble metal catalyst (such as palladium) and a promoter (such as barium sulfate or nickel oxide).

However, the conventional processes cannot always produce coumarin derivatives in high yields. It is therefore, desirable to provide a process for producing a coumarin derivative in a high yield.

We have developed a process for producing coumarin derivatives in good yields at low cost, by use of a novel catalyst and promoter for use in the ring formation and dehydrogenation reaction.

The present invention provides a process for producing a coumarin derivative represented by formula (II): 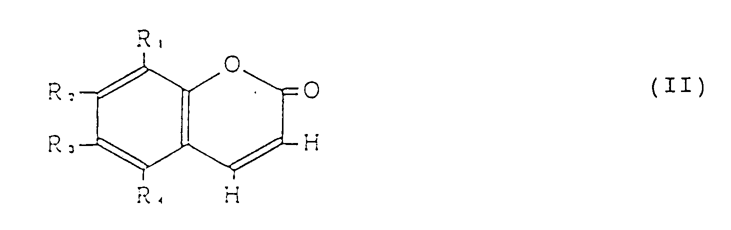
wherein R₁ to R₄ each represents a hydrogen atom, a methyl group or an ethyl group, provided that at least two of R₁ to R₄ each represents a hydrogen atom,
the process comprising the step of: subjecting to a ring formation and dehydrogenation reaction a 3-(2-cyclohexanoyl)propionic acid ester derivative represented by formula (I): 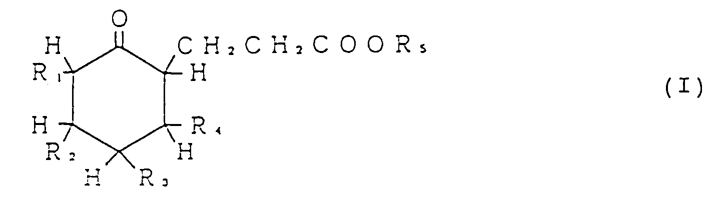
wherein R₁ to R₄ are as defined above, and R₅ represents an alkyl group having 1 to 4 carbon atoms,
the ring formation and dehydrogenation reaction being conducted by use of (1) a catalyst comprising a carrier having supported thereon palladium and either chromium oxide or chromium hydroxide, or
(2) a catalyst comprising a carrier having supported thereon palladium or comprising a carrier having supported thereon palladium and either chromium oxide or chromium hydroxide, in the presence of a promoter which is at least one of magnesium trisilicate, zirconia, metallic chromium and metallic tungsten.

The 3-(2-cyclohexanoyl)propionic acid ester derivative used in the present invention is represented by formula (I) given above. Examples of this compound include:
methyl 3-(2-cyclohexanoyl)propionate (which is preferred),
ethyl 3-(2-cyclohexanoyl)propionate,
butyl 3-(2-cyclohexanoyl)propionate,
methyl 3-(2-cyclohexanoyl-3-methyl)propionate,
methyl 3-(2-cyclohexanoyl-5-methyl)propionate,
propyl 3-(2-cyclohexanoyl-4-ethyl)propionate,
propyl 3-(2-cyclohexanoyl-3,4-diethyl)propionate,
propyl 3-(2-cyclohexanoyl-3,4-dimethyl)propionate,
methyl 3-(2-cyclohexanoyl-3,5-diethyl)propionate, and
methyl 3-(2-cyclohexanoyl-3-ethyl-6-methyl)propionate.

The catalyst used in the present invention is a heterogeneous metal catalyst which comprises palladium supported on a carrier, or comprises palladium and either chromium oxide or chromium hydroxide, both of which are supported on a carrier. The carrier preferably is at least one element or compound thereof of Group IIA, IIIA and IVA of the Periodic Table, such as carbon, alumina, silica gel or barium sulfate.

These catalysts may be prepared by known methods, for example, by the impregnation-fixation technique as described, e.g., in Shokubai Jikken Manual (Catalyst Experiment Manual), edited by Shokubai Gakkai, published by Maki Shoten, Japan, in which a carrier is impregnated with a metal compound and the resulting carrier is subjected to hydrogen reduction at a high temperature. However, a commercially available catalyst may also be used as it is.

The amount of the catalyst used for the ring formation and dehydrogenation reaction is generally from 0.1 to 5% by weight, preferably from 0.3 to 2% by weight, based on the amount of the 3-(2-cyclohexanoyl)propionic acid ester derivative, since too small an amount of the catalyst results in very low reactivity, whereas too large an amount thereof results not only in an increased amount of by-products because of too high reactivity, but also in an increased cost.

In the case of using the catalyst comprising palladium and chromium oxide or chromium hydroxide supported on a carrier, the amount by weight of the chromium oxide or hydroxide is generally from 1 to 20%, preferably from 5 to 15% based on the amount of the palladium.

Together with a chromium oxide or hydroxide type of catalyst, at least one of magnesium trisilicate, zirconia, metallic chromium and metallic tungsten may be used as a promoter for assisting the ring formation and dehydrogenation reaction. Such a commercially available substance may be used as it is without any treatment.

The amount of the promoter used is generally about from 0.01 to 3% by weight, preferably about from 0.05 to 2% by weight, based on the amount of the cyclohexanoyl propionic acid ester derivative.

The ring formation and dehydrogenation reaction of said propionic acid ester derivative may be conducted generally at from 100 to 350°C, preferably at from 230 to 280°C. Temperature outside the above range are not preferred because too low a temperature results in low reactivity, while a temperature exceeding 350°C tends to cause the starting material and/or the product to decompose.

The propionic ester derivative may be used dissolved in a solvent such as phenyl ether, benzyl ether, methyl α-naphthyl ether, ethylnaphthalene, dimethylbiphenyl, dodecane, tetradecane, tetralin, acetophenone, phenyl propyl ketone, methyl benzoate or dimethyl glutamate.
The amount of solvent is generally from 0.5 to 10 times, preferably from 1 to 7 times, the amount of said propionic acid derivative.

The ring formation and dehydrogenation reaction can be carried out by introducing the 3-(2-cyclohexanoyl)propionic acid ester derivative and either the catalyst or both the catalyst and the promoter, and then heating the resulting mixture, together with a solvent if required, at a predetermined temperature, generally for about 5 hours to about 50 hours. The reaction is generally carried out in an inert gas atmosphere under ordinary pressure. As a result, 3,4-dihydrocoumarin derivative is obtained in a yield of about from 30 to 45%, and a coumarin derivative is obtained in a yield of about from 30 to 40%. Besides these compounds, o-ethylphenols, methyl dihydrocinnamic acid esters and octahydrocoumarins are formed as by-products

By means of the process of the invention, coumarin derivatives can be produced in higher yields than by conventional processes.

The invention will be illustrated by the following examples, wherein percentages are by weight.

### EXAMPLES

In a four-necked flask, there were mixed 200 or 300 g (as shown in the Table) of methyl 3-(2-cyclohexanoyl)propionate (MCP) and 2 or 3 g of a catalyst consisting of active carbon as support and 5% by weight of palladium and optionally another catalyst component and one or two promoters, as shown in the Table. The resulting mixture in the flask was heated at 240°C for 10 hours with stirring at a speed of 300 r.p.m. in a nitrogen atmosphere. Thereafter the mixture was heated at 260°C and maintained at this temperature for 5 hours (except in Ex. 5 when this heating was at 255°C for 1 hour) and was then heated to 270°C and was maintained thereat for 15 hours.

After completion of the reaction, the resulting reaction mixture was filtered to remove the catalyst and promoter and then analysed by gas chromatography. The yields and conversions to coumarin and 3,4-dihydrocoumarin, based on the MCP, are shown in the Table.

In Comparative Examples 1 to 4, the promoters used were not according to the invention and the yields of coumarin were lower.

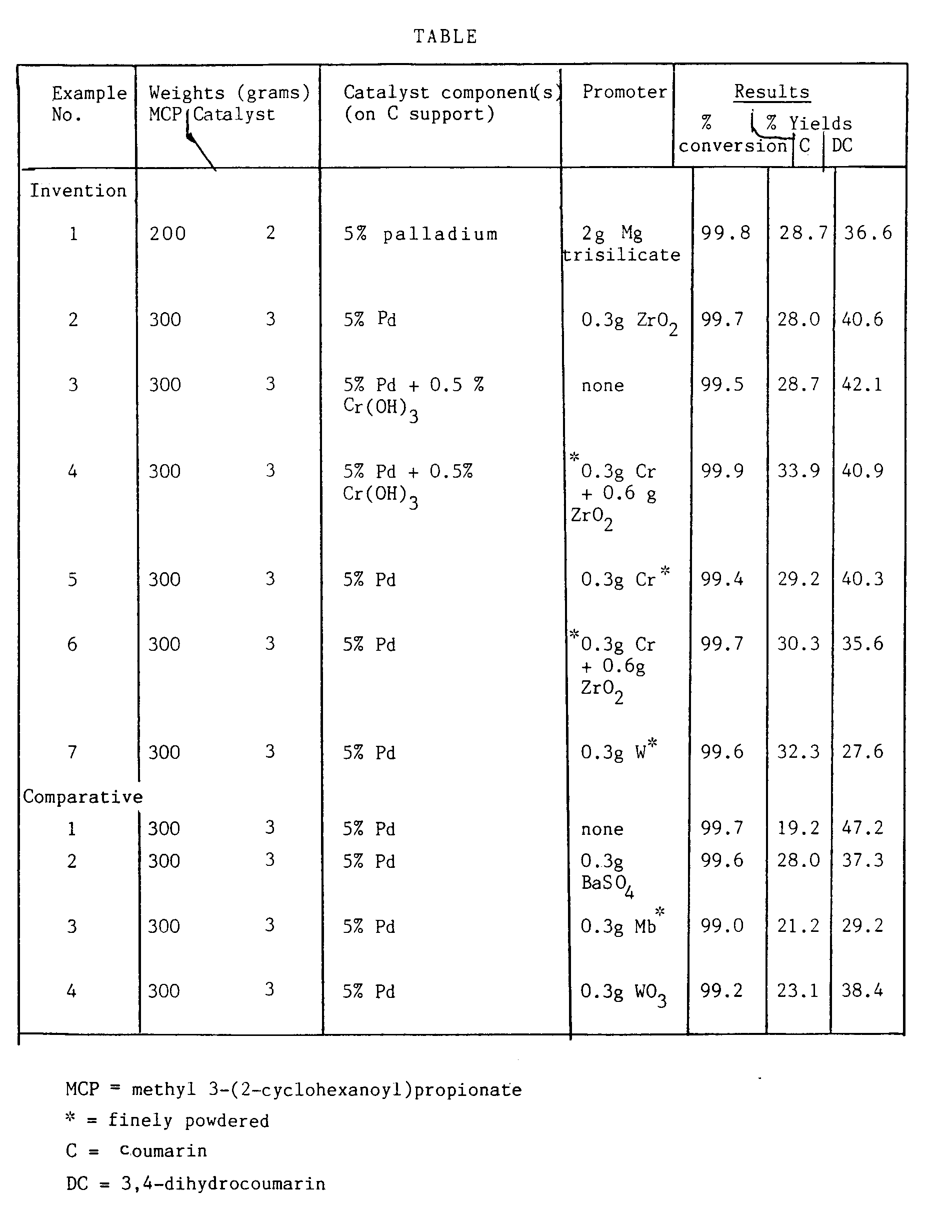

## Claims

1. A process for producing a coumarin derivative represented by formula (II): 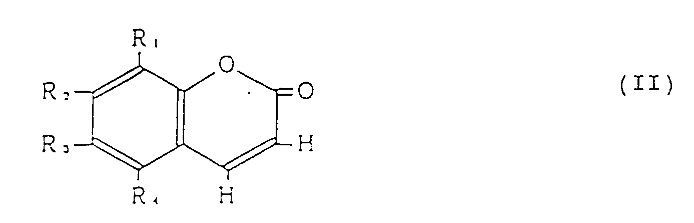 wherein R₁ to R₄ each represents a hydrogen atom, a methyl group or an ethyl group, provided that at least two of R₁ to R₄ each represents a hydrogen atom,
said process comprising subjecting a 3-(2-cyclohexanoyl)propionic acid ester derivative represented by the formula (I): 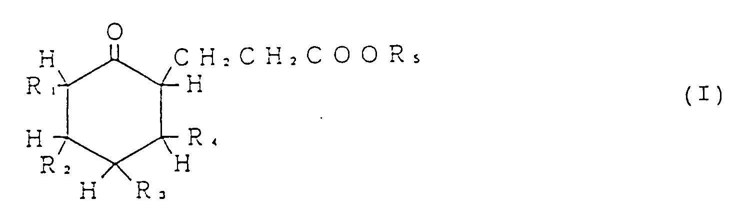 wherein R₁ to R₄ are as defined above and R₅ represents an alkyl group having 1 to 4 carbon atoms, to ring formation and dehydrogenation reaction by use of a catalyst comprising a carrier having supported thereon palladium and either chromium oxide or chromium hydroxide.

2. A process for producing a coumarin derivative as defined in Claim 1, which is a modification of the process of Claim 1 wherein the catalyst used comprises a carrier having supported thereon palladium or comprises a carrier having supported thereon palladium and either chromium oxide or chromium hydroxide, in the presence of a promoter which is at least one of magnesium trisilicate, zirconia, metallic chromium and metallic tungsten.

3. A process as claimed in Claim 1 or 2, wherein said 3-(2-cyclohexanoyl)propionic acid ester derivative is methyl 3-(2-cyclohexanoyl)propionate.

4. A process as claimed in Claim 1, 2 or 3, wherein said carrier is selected from carbon, alumina, silica gel and barium sulfate.

5. A process as claimed in any preceding claim, wherein the amount of said chromium oxide or chromium hydroxide supported on the carrier is 1 to 20 wt% based on the amount of palladium.

6. A process as claimed in any preceding claim, wherein said catalyst is used in an amount of 0.1 to 5 wt% based on the amount of said cyclohexanoyl propionic acid ester derivative.

7. A process as claimed in any preceding claim, wherein said promoter is used in an amount of 0.01 to 3 wt% based on the amount of said cyclohexanoyl propionic acid ester derivative.

8. A process as claimed in any preceding claim, wherein said ring formation and dehydrogenation reaction is conducted at a temperature of from 100 to 350°C.

## Patentansprüche

1. Verfahren zur Herstellung eines durch die Formel (II) beschriebenen Cumarinabkömmlings: 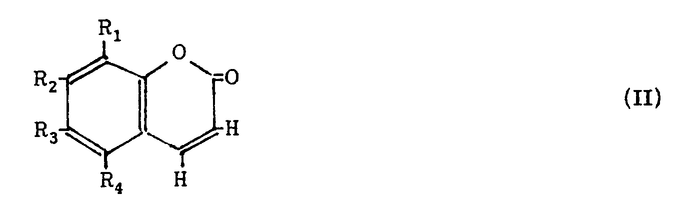 worin jede Gruppe R₁ bis R₄ ein Wasserstoffatom, eine Methylgruppe oder eine Ethylgruppe darstellt, sofern zumindest zwei davon je ein Wasserstoffatom darstellen,
dadurch gekennzeichnet, dass ein 3-(2-Cyclohexanoyl)propionsäureesterabkömmling der Formel (I) 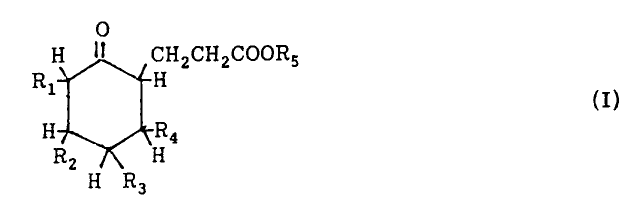 worin R₁ bis R₄ wie oben definiert sind, während R₅ eine Alkylgruppe mit ein bis vier Kohlenstoffatomen darstellt,
einer Ringschluss- und Dehydrierungsreaktion unter Benutzung eines Katalysators unterworfen wird, der einen Träger und auf diesem Träger Palladium und entweder Chromoxid oder Chromhydroxid umfasst.

2. Verfahren zur Herstellung eines Cumarinabkömmlings, wie er in Anspruch 1 definiert ist, das eine Abwandlung des Verfahrens von Anspruch 1 darstellt, in der der benutzte Katalysator einen Träger und auf diesem Träger Palladium oder einen Träger und auf diesem Palladium und entweder Chromoxid oder Chromhydroxid darstellt, wobei zumindest ein aus der Gruppe von Magnesiumtrisilikat, Zironiumdioxid, metallischem Chrom oder metallischem Wolfram ausgewählter Aktivator zugegen ist.

3. Verfahren gemäss Ansprüchen 1 oder 2, in dem der benannte 3-(2-Cyclohexanoyl)propionsäureesterabkömmlingethyl-3-(2-cyclohexanoyl)propionat ist.

4. Verfahren gemäss Ansprüchen 1, 2 oder 3, in dem der benannte Träger aus der Gruppe von Kohle, Aluminiumoxid, Silicagel und Bariumsulfat ausgewählt ist.

5. Verfahren nach einem der vorangehenden Ansprüche, in dem die Menge des benannten, auf dem Träger befindlichen Chromoxids oder Chromhydroxids 1 bis 20 Gewichtsprozent bezogen auf die Palladiummenge beträgt.

6. Verfahren nach einem der vorangehenden Ansprüche, in dem der benannte Katalysator in einer 0,1 bis 5 Gewichtsprozent bezogen auf die Menge des benannten Cyclohexanoylpropionsäureesterabkömmlings betragenden Menge eingesetzt wird.

7. Verfahren nach einem der vorangehenden Ansprüche, in dem der benannte Aktivator in einer 0,01 bis 3 Gewichtsprozent bezogen auf den Menge des benannten Cyclohexanoylpropionsäureesterabkömmlings betragenden Menge eingesetzt wird.

8. Verfahren nach einem der vorangehenden Ansprüche, in dem die benannte Ringschluss- und Dehydrierungsreaktion bei einer Temperatur zwischen 100 und 350 °C durchgeführt wird.

## Revendications

1. Procédé pour produire un dérivé de la coumarine représenté par la formule (II) : 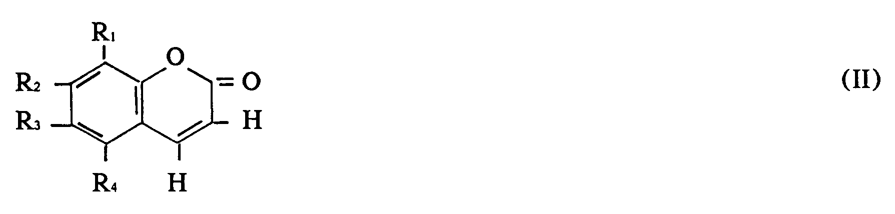 dans laquelle R₁ à R₄ représentent chacun un atome d'hydrogène, un groupe méthyle ou un groupe éthyle, à condition qu'au moins deux d'entre R₁ à R₄ représentent chacun un atome d'hydrogène,
ledit procédé consistant à soumettre un dérivé ester d'acide 3-(2-cyclohexanoyl)propionique représenté par la formule (I) : 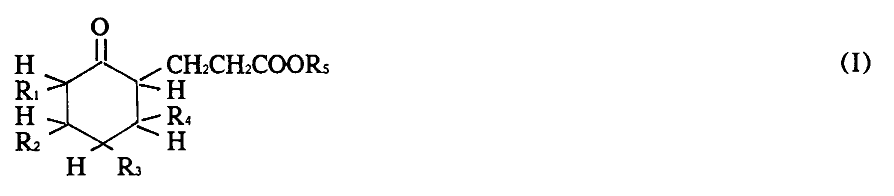 dans laquelle R₁ à R₄ sont comme défini ci-dessus et R₅ représente un groupe alkyle ayant de 1 à 4 atomes de carbone, à une réaction de cyclisation et de déshydrogénation, en utilisant un catalyseur contenant un support portant du palladium et soit de l'oxyde de chrome, soit de l'hydroxyde de chrome.

2. Procédé pour produire un dérivé de la coumarine comme défini dans la revendication 1, qui est une modification du procédé de la revendication 1, où le catalyseur utilisé comprend un support portant du palladium ou un support portant du palladium plus soit de l'oxyde de chrome, soit de l'hydroxyde de chrome, en présence d'un promoteur qui est au moins un d'entre le trisilicate de magnésium, la zircone, le chrome métal et le tungstène métal.

3. Procédé selon la revendication 1 ou 2, où ledit dérivé ester d'acide 3-(2-cyclohexanoyl)propionique est le 3-(2-cyclohexanoyl)propionate de méthyle.

4. Procédé selon la revendication 1, 2 ou 3, où ledit support est choisi parmi le carbone, l'alumine, le gel de silice et le sulfate de baryum.

5. Procédé selon l'une quelconque des revendications précédentes, où la quantité dudit oxyde de chrome ou hydroxyde de chrome porté par le support est égale à 1 - 20% en poids, sur la base de la quantité de palladium.

6. Procédé selon l'une quelconque des revendications précédentes, où ledit catalyseur est utilisé en une quantité allant de 0,1 à 5% en poids, sur la base de la quantité dudit dérivé ester d'acide cyclohexanoylpropionique.

7. Procédé selon l'une quelconque des revendications précédentes, où ledit promoteur est utilisé en une quantité allant de 0,01 à 3% en poids, sur la base de la quantité dudit dérivé ester d'acide cyclohexanoylpropionique.

8. Procédé selon l'une quelconque des revendications précédentes, où ladite réaction de cyclisation et de déshydrogénation est effectuée à une température comprise entre 100 et 350°C.
